(19) Europäisches Patentamt
European Patent Office
Office européen des brevets

(11) EP 4 190 330 B1

(12) EUROPEAN PATENT SPECIFICATION

(45) Date of publication and mention of the grant of the patent:
**10.09.2025 Bulletin 2025/37**

(21) Application number: **21849180.1**

(22) Date of filing: **30.07.2021**

(51) International Patent Classification (IPC):
***A61K 31/4535*** *(2006.01)* ***A61P 3/04*** *(2006.01)*
***A23L 33/10*** *(2016.01)*

(52) Cooperative Patent Classification (CPC):
**A61P 3/04; A23L 33/10; A61K 31/4535**

(86) International application number:
**PCT/KR2021/010001**

(87) International publication number:
**WO 2022/025716 (03.02.2022 Gazette 2022/05)**

(54) **USE OF 2,3,5-SUBSTITUTED THIOPHENE COMPOUND FOR PREVENTING, ALLEVIATION, OR TREATING MASTOCYTOSIS**

VERWENDUNG VON 2,3,5-SUBSTITUIERTER THIOPHENVERBINDUNG ZUR VORBEUGUNG, LINDERUNG ODER BEHANDLUNG VON MASTOZYTOSE

UTILISATION D'UN COMPOSÉ THIOPHÈNE 2,3,5-SUBSTITUÉ POUR PRÉVENIR, SOULAGER OU TRAITER UNE MASTOCYSTOSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.07.2020 KR 20200095988**

(43) Date of publication of application:
**07.06.2023 Bulletin 2023/23**

(73) Proprietors:
- **Pharos Ibio Co., Ltd.**
  **Gyeonggi-do 14059 (KR)**
- **Korea Institute of Science and Technology**
  **Seoul 02792 (KR)**

(72) Inventors:
- **SIM, Tae Bo**
  **Seoul 02792 (KR)**
- **CHOI, Seung Hye**
  **Seoul 02792 (KR)**
- **CHO, Han Na**
  **Seoul 02792 (KR)**
- **HUR, Woo Young**
  **Seoul 02792 (KR)**
- **SONG, Chi Man**
  **Seoul 02792 (KR)**
- **NAM, Ky Youb**
  **Goyang-si Gyeonggi-do 10360 (KR)**
- **YOON, Jeong Hyeok**
  **Yongin-si Gyeonggi-do 16902 (KR)**

(74) Representative: **Weickmann & Weickmann PartmbB**
**Postfach 860 820**
**81635 München (DE)**

(56) References cited:
**EP-A1- 3 418 275** **KR-A- 20060 127 127**
**KR-A- 20090 130 345** **KR-A- 20170 096 599**
**US-A1- 2006 063 773**

- **GALLOGLY MOLLY M. ET AL: "Midostaurin: a novel therapeutic agent for patients with FLT3-mutated acute myeloid leukemia and systemic mastocytosis", THERAPEUTIC ADVANCES IN HEMATOLOGY, vol. 8, no. 9, 19 August 2017 (2017-08-19), GB, pages 245 - 261, XP093179559, ISSN: 2040-6207, DOI: 10.1177/2040620717721459**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 190 330 B1

- PIRIS-VILLAESPESA MIGUEL ET AL: "Systemic Mastocytosis: Following the Tyrosine Kinase Inhibition Roadmap", FRONTIERS IN PHARMACOLOGY, vol. 11, 14 April 2020 (2020-04-14), CH, pages 443, XP093179931, ISSN: 1663-9812, DOI: 10.3389/fphar.2020.00443
- KE HENGNING, KAZI JULHASH U., ZHAO HUI, SUN JIANMIN: "Germline mutations of KIT in gastrointestinal stromal tumor (GIST) and mastocytosis", CELL & BIOSCIENCE, vol. 6, no. 1, 1 December 2016 (2016-12-01), pages 55, XP055891243, DOI: 10.1186/s13578-016-0120-8

## Description

### Technical Field

The present invention relates to the use of a 2,3,5-substituted thiophene compound for the prevention, amelioration or treatment of mastocytosis.

### Background Art

Mastocytosis is a rare disease in which mast cells are abnormally accumulated in internal organs such as skin, bone marrow, liver, spleen and lymph nodes. A study in the UK reported that the incidence of systemic mastocytosis is 2 in 300,000 per year.

Mast cells are immune cells that are a major cause of allergy. On the surface of mast cells, there are surface factors to which IgE-type antibodies may bind. Unlike other antibodies that detach relatively immediately after binding to a substrate, IgE hardly detach after binding to mast cells. In this case, when a substance (i.e., an allergen) that binds to IgE enters the human body and binds to IgE, mast cells having IgE bound thereto are activated. These activated mast cells induce an allergic reaction by secreting the neurotransmitter histamine to the outside. In patients with mastocytosis that begins in adulthood, mastocytosis affects not only the skin but also internal organs, whereas if the disease begins in childhood, distinct symptoms thereof appear on the skin, but very mild symptoms may appear in body organs or the disease may not affect organs. If symptoms of this disease appear in the bone marrow or internal organs of the body, the disease is called "systemic mastocytosis". In most patients, the disease tends to progress slowly, but in some patients diagnosed with mastocytosis, hematologic diseases such as myelodysplastic disorder or myeloproliferative disorder are also found. The progression and prognosis of mastocytosis is determined in relation to these hematologic diseases. More severe mastocytosis and mast cell leukemia occur in very few patients. Mastocytosis occurs in equal proportions in males and females, and the childhood-onset form of mastocytosis mainly begins around 2 years of age.

In the case of adult-onset mastocytosis, excessive activity of mast cell growth factor (c-kit) receptors due to genetic mutations causes abnormal mast cells. In addition, these mutations are considered responsible for the accumulation of mast cells in specific tissues. Symptoms are caused by substances such as histamine, heparin, and prostaglandin D2, which are mediators released from mast cells. Histamine is a natural chemical substance and is released during a normal allergic reaction. This causes itching, wheezing, vasodilation, and excessive secretion of stomach acid. It has been reported that, when a mutation in the KIT gene gains a function, systemic mastocytosis occurs. KIT is a tyrosine kinase receptor involved in hematopoiesis, mast cell development and function, gametogenesis and melanogenesis. The KIT gene is located on the long arm of chromosome 4 (4q12), and encodes a class III receptor tyrosine kinase, KIT. KIT D816V is the most prevalent KIT mutation in mastocytosis and occurs in more than 90% of the cases that fulfill the World Health Organization diagnostic criteria for systemic mastocytosis. However, its exact pathogenesis is still unknown.

The TK inhibitor imatinib (STI571), which is widely used in clinical hematology, has recently been found to counteract the growth of neoplastic mast cells exhibiting wild type (wt) KIT or the rarely occurring F522C-mutated variant of KIT (Akin C, Brockow K, D'Ambrosio C, et al. Effects of tyrosine kinase inhibitor STI571 on human mast cells bearing wild-type or mutated forms of c-kit. Exp Hematol 2003; 31:686-692.; Ma Y, Zeng S, Metcalfe DD, et al. The c-KIT mutation causing human mastocytosis is resistant to STI571 and other KIT kinase inhibitors; kinases with enzymatic site mutations show different inhibitor sensitivity profiles than wild-type kinases and those with regulatory type mutations. Blood 2002; 99:1741-1744.; Frost MJ, Ferrao PT, Hughes TP, Ashman LK. Juxtamembrane mutant V560GKit is more sensitive to Imatinib (STI571) compared with wild-type c-kit whereas the kinase domain mutant D816VKit is resistant. Mol Cancer Ther. 2002; 1 : 1115- 1124.; Akin C, Fumo G, Yavuz AS, Lipsky PE, Neckers L, Metcalfe DD. A novel form of mastocytosis associated with a transmembrane c-kit mutation and response to imatinib. Blood. 2004; 103:3222-3225). However, imatinib failed to inhibit the growth of neoplastic mast cells with the c-KIT mutation D816V. Therefore, there is a need to develop a novel TK inhibitor that inhibits the growth of neoplastic mast cells in systemic mastocytosis by inhibiting KIT D816V. EP3418275 relates to a novel 2,3,5-substituted thiophene compounds including (S)-5-((3-fluorophenyl)ethynyl)-N -(piperidin-3-yl)-3-ureidothiophene-2-carboxamide. These compounds have inhibitory activity against kinases for suppressing proliferation of FLT3-ITD holding leukemia cell line and Ba/F3 cell line.

Gallogly MM. et al. (Therapeutic Advances in Hematology, 2017, 8(9):245-261) discloses midostaurin for treating FLT3-mutated systemic mastocytosis.

US2006063773 discloses (hydrazido) (amino)thiophene compounds useful for the treatment of hyperproliferative disorders, including mastocytosis/mast cell leukemia and others.

Accordingly, the present inventors have conducted studies to develop a novel substance that may be used for the treatment of mastocytosis, thereby completing the present invention.

### DISCLOSURE

## Technical Problem

The present invention relates to a compound represented by the following Formula 1 or a pharmaceutically acceptable salt thereof for use in preventing or treating mastocytosis. In an embodiment, the mastocytosis is any one disease selected from the group consisting of mast cell leukemia, systemic mastocytosis, and invasive systemic mastocytosis. In a further embodiment, the mastocytosis is caused by substitution of valine or histidine for aspartic acid at amino acid position 816 of c-kit protein. In a further embodiment, the mastocytosis is caused by at least one substitution selected from the group consisting of: substitution of valine for glycine at amino acid position 560 of c-kit protein; substitution of proline for alanine at amino acid position 829 of c-kit protein; substitution of proline for leucine at amino acid position 576 of c-kit protein; substitution of aspartic acid for valine at amino acid position 559 of c-kit protein; substitution of isoleucine for threonine at amino acid position 670 of c-kit protein; and substitution of alanine for valine at amino acid position 654 of c-kit protein. In a further embodiment, the compound is contained in a pharmaceutical composition or food composition.

The references to the methods of treatment of this description are to be interpreted as references to compounds, pharmaceutical compositions and medicaments of the present invention for use in in a method for treatment of the human (or animal) body by therapy (or for diagnosis).

An object of the present invention is to provide a pharmaceutical composition for use in preventing or treating mastocytosis containing a compound represented by the following Formula 1 or a pharmaceutically acceptable salt thereof:

[Formula 1]

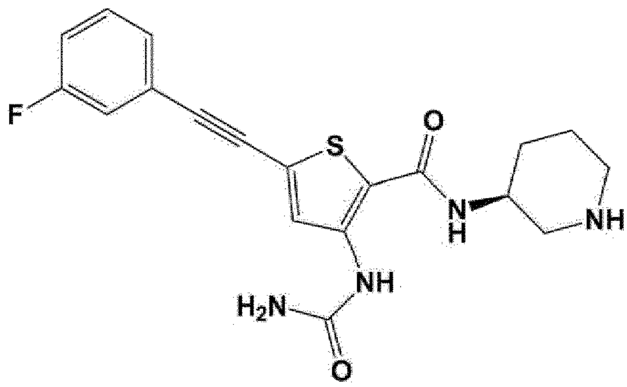

Another object of the present invention is to provide a food composition for use in preventing or ameliorating mastocytosis containing a compound represented by the following Formula 1 or a pharmaceutically acceptable salt thereof:

[Formula 1]

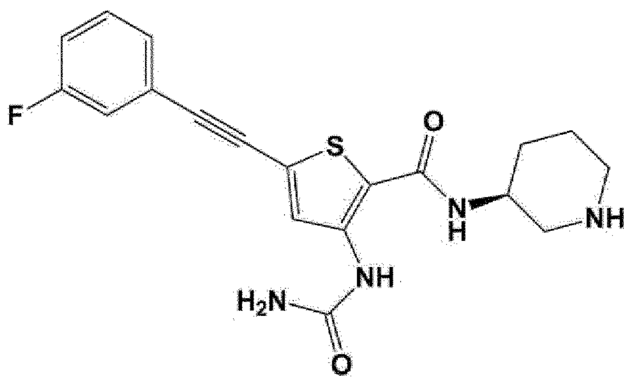

Technical **Solution**

One aspect of the present invention provides a pharmaceutical composition for use in preventing or treating mastocytosis containing a compound represented by the following Formula 1 or a pharmaceutically acceptable salt thereof:

[Formula 1]

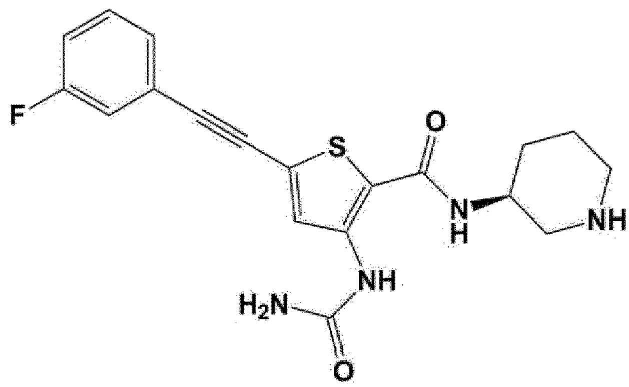

Another aspect of the present invention provides a food composition for use in preventing or ameliorating mastocytosis containing a compound represented by the following Formula 1 or a pharmaceutically acceptable salt thereof:

[Formula 1]

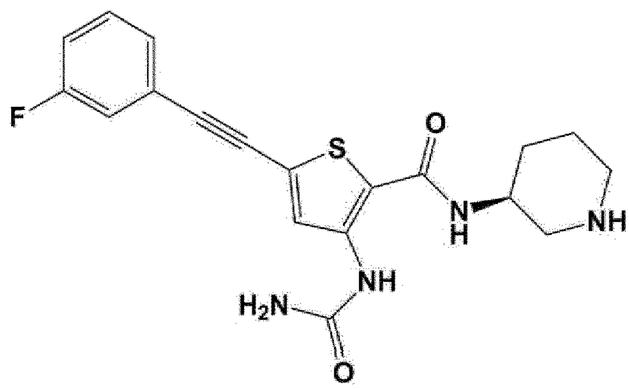

**Advantageous Effects**

The composition containing a 2,3,5-substituted thiophene compound according to one embodiment of the present invention has excellent inhibitory activity against the growth of mastocytosis cells and against an inflammatory response caused by mastocytosis, and thus may be effectively used for the prevention, amelioration or treatment of mastocytosis.

**Brief Description of Drawings**

FIG. 1 shows a mutation of aspartic acid (D)-to-valine (V) substitution at amino acid position 816 in the amino acid sequence of c-kit protein.

FIG. 2 depicts photographs showing the inhibitory activities of PHI-101 (A) according to an embodiment of the present

invention, sunitinib (B) and dasatinib (C) against signaling in the c-kit D816V Ba/F3 cell line at various treatment concentrations.

FIG. 3 depicts graphs showing the results of Annexin V-PI FACS analysis of the c-kit D816V Ba/F3 cell line treated with various concentrations of dimethyl sulfoxide (DMSO) (A) as a negative control, sunitinib (B) and (C), and PHI-101 (D), (E) and (F) according to one embodiment of the present invention.

FIG. 4 depicts graphs showing the results of cell cycle FACS analysis of the c-kit D816V Ba/F3 cell line treated with dimethyl sulfoxide (DMSO) (A) as a negative control, sunitinib (B), and PHI-101 (C) according to one embodiment of the present invention.

FIG. 5 is a graph showing the population at each cell cycle phase of the c-kit D816V Ba/F3 cell line treated with dimethyl sulfoxide (DMSO) (A) as a negative control, sunitinib (B), and PHI-101 (C) according to one embodiment of the present invention.

FIG. 6 is a photograph showing the inhibitory activities of PHI-101 according to one embodiment of the present invention, imatinib and sunitinib against signaling in the HMC-1.2 cell line at various treatment concentrations.

FIG. 7 depicts graphs showing the results of Annexin V-PI FACS analysis performed to evaluate the apoptotic effects of PHI-101 according to one embodiment of the present invention and imatinib against the HMC-1.2 cell line at various treatment concentrations.

FIG. 8 is a photograph showing the results of Western blot analysis performed to evaluate the apoptotic effects of PHI-101 according to one embodiment of the present invention, imatinib and sunitinib against the HMC-1.2 cell line at various treatment concentrations.

FIG. 9 is a photograph comparing the status of mastocytosis mouse models on day 21 after oral administration of each of PHI-101 according to one embodiment of the present invention, imatinib and sunitinib.

FIG. 10 is a graph comparing the tumor growth inhibitory activities of PHI-101 according to one embodiment of the present invention, imatinib and sunitinib in mastocytosis mouse models after oral administration.

FIG. 11 depict graphs showing tumor volumes in mastocytosis mouse models on day 0 (A) and day 21 (B) after oral administration of each of PHI-101 according to one embodiment of the present invention, imatinib and sunitinib.

FIG. 12 depicts graphs showing tumor volumes (A) and tumor weights (B) (percentages relative to a control) in mastocytosis mouse models on day 21 (B) after oral administration of each of PHI-101 according to one embodiment of the present invention, imatinib and sunitinib.

FIG. 13 is a photograph showing the spleens in mastocytosis mouse models on day 21 (B) after oral administration of each of PHI-101 according to one embodiment of the present invention, imatinib and sunitinib.

FIG. 14 depicts graphs showing spleen/body weight ratio (A) and spleen/body weight ratio (B) (percentages relative to a control) in mastocytosis mouse models on day 21 (B) after oral administration of each of PHI-101 according to one embodiment of the present invention, imatinib and sunitinib.

## Best Mode

One aspect of the present invention provides a pharmaceutical composition for use in preventing or treating mastocytosis containing a compound represented by the following Formula 1 or a pharmaceutically acceptable salt thereof:

[Formula 1]

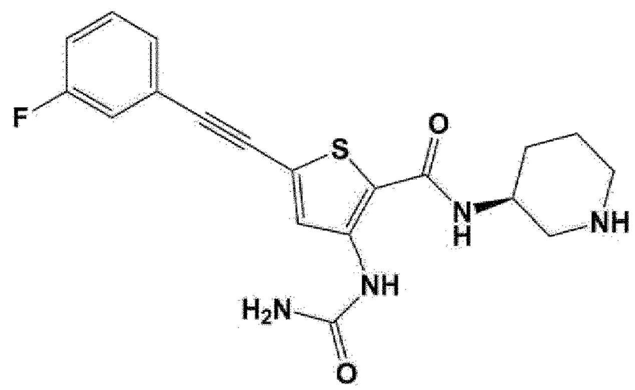

In one embodiment of the present invention, the mastocytosis may be any one disease selected from the group

consisting of mast cell leukemia, systemic mastocytosis, and invasive systemic mastocytosis.

In one embodiment of the present invention, the mastocytosis may be caused by substitution of valine or histidine for aspartic acid at amino acid position 816 of c-kit protein.

In one embodiment of the present invention, the mastocytosis may be caused by at least one substitution selected from the group consisting of substitution of valine for glycine at amino acid position 560 of c-kit protein, substitution of proline for alanine at amino acid position 829 of c-kit protein, substitution of proline for leucine at amino acid position 576 of c-kit protein, substitution of aspartic acid for valine at amino acid position 559 of c-kit protein, substitution of isoleucine for threonine at amino acid position 670 of c-kit protein, and substitution of alanine for valine at amino acid position 654 of c-kit protein

Another aspect of the present invention provides a food composition for use in preventing or ameliorating mastocytosis containing a compound represented by the following Formula 1 or a pharmaceutically acceptable salt thereof:

[Formula 1]

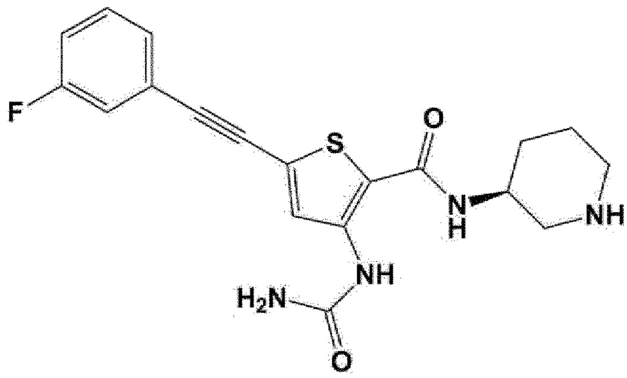

**Mode for Invention**

Hereinafter, the present invention will be described in detail with reference to one or more examples. However, these examples are for illustrating the present invention, and the scope of the present disclosure is not limited by these examples.

**Example 1. Evaluation of inhibitory activity of** PHI-101 **against c-kit mutant cell line**

*1-1. Evaluation of inhibitory activity against growth of c-kit mutant cell line*

The inhibitory activity of the compound represented by Formula 1 (hereinafter referred to as "PHI-101") against a c-kit mutant cell line was measured.

[Formula 1]

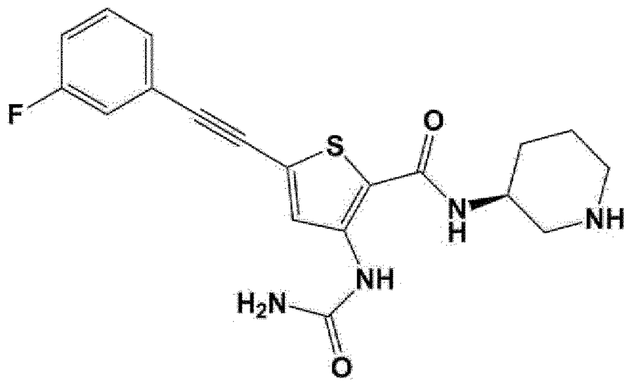

Specifically, 100 ul of a Ba/F3 cell line with a c-kit D816V mutation (FIG. 1) was added to each well of a 96-well plate at a concentration of 10,000 cells/100 ul. After 4 hours, 100 μl of the Ba/F3 cell line was treated with 0.5 ul of each of control compounds and PHI-101 (10-point 1:3 serial dilutions) to a final concentration of up to 50 μM, and cultured at 37°C under 5% $CO_2$ for 72 hours. As the control compounds, imatinib and sunitinib which are c-kit inhibitors were used. After culture, the cells were counted using a Celltiter glo assay kit (Promega), and the 50% growth inhibition values ($GI_{50}$, μM) of the control compounds and PHI-101 were measured.

As a result, it was confirmed that PHI-101 exhibited higher inhibitory activity against the c-kit D816V Ba/F3 mutant cell line than the control compounds, and exhibited higher selectivity for the c-kit D816V Ba/F3 mutant cell line than for the parental Ba/F3 cell line (Table 1).

[Table 1]

| | $GI_{50}$ (μM) | | |
|---|---|---|---|
| | PHI-101 | Imatinib | Sunitinib |
| cKIT D816V Ba/F3 | 0.912 | 11.48 | 2.647 |
| Parental Ba/F3 | 5.010 | 12.73 | 13.32 |

*1-2. Evaluation of inhibitory activity against c-kit phosphorylation and downstream signaling*

The inhibitory activities of control compounds and PHI-101 against c-kit phosphorylation and downstream signaling (p-STAT3, p-ERK1/2) in the c-kit D816V mutant Ba/F3 cell line were measured, and as the control compounds, sunitinib and dasatinib were used.

Specifically, the c-kit D816V mutant Ba/F3 cell line was dispensed at a concentration of 1 x $10^6$ cells/ml, treated with each of the control compounds and PHI-101 at concentrations of 0.1, 1 and 10 μM, and then cultured for 2 hours. Next, lysates of the cells were obtained using lysis buffer (50 mM Tris-HCl pH 7.5, 1% NP40, 1 mM EDTA, 150 mM NaCl, 5 mM, $Na_3VO_4$ and 2.5 mM NaF, and a protease inhibitor cocktail). Equal amounts of the cell lysates were electrophoresed using 8% SDS-PAGE gel at 100 V for 1 hour and 30 minutes, and the separated protein was electrically transferred to nitrocellulose membranes. The membranes were blocked with 5% skim milk at room temperature for 30 minutes, and allowed to react with primary antibodies (diluted 1:5,000), including p-c-kit tyrosine 719 (Y719), p-STAT3 tyrosine 705 (Y705) and p-ERK1/2 threonine 202/tyrosine 204 (T202/Y204) at 4°C for 16 to 20 hours. As a control for comparing the level of expression, anti-β actin (diluted at a ratio of 1:10,000) was used as a primary antibody. Thereafter, the membranes were washed 3 times with TBST (Tris-buffered saline, 0.1% Tween 20) for 5 minutes each time, and then incubated with an HRP-conjugated anti-rabbit secondary antibody (diluted at a ratio of 1:10,000) at room temperature for 1 hour. Next, the membranes were washed three times with TBST for 5 minutes each time and treated with a chemiluminescent substrate reagent, and the expression level of the protein was measured by detecting luminescence with an X-ray film in the dark.

As a result, it was confirmed that PHI-101 showed higher inhibitory activity against p-c-kit (Y719) phosphorylation than the control compounds, and showed downstream signaling (p-STAT3, p-ERK1/2) inhibitory activity similar to those of the control compounds (FIG. 2).

*1-3. Evaluation of apoptotic effect*

The c-kit D816V mutant Ba/F3 cell line was treated with PHI-101, and then the apoptotic effect of PHI-101 was analyzed.

Specifically, the c-kit D816V mutant Ba/F3 cell line was adjusted to a concentration of 1 x $10^6$ cells/ml and treated with each of PHI-101 at concentrations of 0.1, 1 and 10 μM and the control compound sunitinib at concentrations of 1 and 10 μM for 24 hours. Thereafter, the medium was removed and the cells were washed once with 1 ml PBS. The PBS was removed by centrifugation at 1,000 rpm for 5 minutes. The cells were dissolved in 100 μl of PBS containing $Ca^{2+}$ and $Mg^{2+}$ and allowed to react with 5 μl of Annexin V for 20 minutes. After completion of the reaction, 400 μl of PBS containing $Ca^{2+}$ and $Mg^{2+}$ was added, and 5 μl of propidium iodide (PI) was added, and then the degree of apoptotic cell death was analyzed by FACS (fluorescence activated cell sorter) assay using a flow cytometer (FACS-C6, BD).

As a result, it was confirmed that PHI-101 strongly induced apoptosis even at a concentration of 0.1 μM, and increased apoptosis in a concentration-dependent manner (FIG. 3).

1-4. *Evaluation of inhibitory effect against cell cycle progression*

The c-kit D816V mutant Ba/F3 cell line was treated with 1 μM of PHI-101, and then the SubG1/GO-1 population was analyzed by cell cycle FACS assay.

Specifically, the c-kit D816V mutant Ba/F3 cell line was adjusted to a concentration of 1 x $10^6$ cells/ml and treated with each of PHI-101 and the control compound sunitinib at a concentration of 1 $\mu$M for 24 hours. Thereafter, the medium was removed, and the c-kit D816V mutant Ba/F3 cell line was fixed in 70% ethanol at 4°C for 24 hours, and then incubated in a PBS buffer solution containing 40 $\mu$g/ml of propidium iodide (PI) and 100 $\mu$g/ml of RNase for 30 minutes to completely stain the total DNA. Cell cycle analysis of the stained cells was performed using a flow cytometer (FACS-C6, BD). The proportion of cells in G0/G1 phase, S phase and G2/M phase was measured, and based on the measurement results, the SubG1/GO-1 population was determined.

As a result, it was confirmed that the SubG1/GO-1 population was increased by treatment with PHI-101, indicating that the cell cycle progression of the c-kit D816V mutant Ba/F3 cell line treated with PHI-101 was inhibited compared to that of the cell line treated with sunitinib (FIGS. 4 and 5).

**Example 2. Evaluation of inhibitory activity of PHI-101 against mastocytosis cell line**

*2-1. Measurement of inhibitory activity against growth of mastocytosis cell line*

The inhibitory activity of PHI-101 against a mastocytosis cell line was measured.

Specifically, for the HMC-1.2 cell line (imatinib-resistant cell line, having c-kit G560V/D816V mutation), the $GI_{50}$ ($\mu$M) values of control compounds and PHI-101 were measured in the same manner as in Example 1-1, and as the control compounds, imatinib, dasatinib, sunitinib and ponatinib were used.

As a result, it was confirmed that PHI-101 exhibited significant inhibitory activity against the HMC-1.2 cell line compared to the control compounds (Table 2).

[Table 2]

| | $GI_{50}$ ($\mu$M) | | | | |
|---|---|---|---|---|---|
| | PHI-101 | Imatinib | Dasatinib | Sunitinib | Ponatinib |
| HMC-1.2 | 0.033 | 17.140 | 0.312 | 1.299 | 0.194 |

*2-2. Evaluation of inhibitory activity against c-kit phosphorylation and downstream signaling*

The inhibitory activities of control compounds and PHI-101 against c-kit phosphorylation and downstream signaling (p-STAT5, p-AKT, p-ERK) in a mastocytosis cell line were measured, and as the control compounds, imatinib and sunitinib were used.

Specifically, inhibitory activities against c-kit phosphorylation and downstream signaling were measured in the same manner as in Example 1-2, except that the HMC-1.2 cell line was treated with each of control compounds at concentrations of 0.1 and 1 $\mu$M and PHI-101 at concentrations of 0.01, 0.1 and 1 $\mu$M, and p-c-kit, p-STAT5, p-AKT and p-ERK each diluted at 1:3000, were used as primary antibodies.

As a result, it was confirmed that PHI-101 decreased c-kit phosphorylation and downstream signaling in a concentration-dependent manner, exhibited significantly high inhibitory activity against downstream signaling compared to the control compounds, and strongly inhibited downstream signaling even at a concentration of 0.1 $\mu$M (FIG. 6).

*2-3. Evaluation of apoptotic effect by Annexin V-PI FACS analysis*

**A** mastocytosis cell line was treated with PHI-101, and the levels of cleaved PARP and cleaved caspase-3, which are apoptosis markers, were analyzed. As a control compound, imatinib was used.

Specifically, the apoptotic effect of PHI-101 against the HMC-1.2 cell line was analyzed in the same manner as in Example 1-3, except that the HMC-1.2 cell line was treated with each of the control compounds at a concentration of 1 $\mu$M and PHI-101 at concentrations of 0.1 and 1 $\mu$M.

As a result, it was confirmed that PHI-101 strongly induced apoptosis even at a concentration of 1 $\mu$M, indicating that PHI-101 had an apoptotic effect against the mastocytosis cell line (FIG. 7).

2-4. *Evaluation of apoptotic effect by Western blot analysis*

**A** mastocytosis cell line was treated with PHI-101, and the levels of cleaved PARP and cleaved caspase-3, which are apoptosis markers, were analyzed. As control compounds, imatinib and sunitinib were used.

Specifically, the apoptotic effect of PHI-101 against the HMC-1.2 cell line was analyzed in the same manner as in Example 1-2, except that the HMC-1.2 cell line was treated with each of the control compounds at a concentration of 1 $\mu$M

and PHI-101 at concentrations of 0.1 and 1 μM, and that cleaved PARP and cleaved caspase-3, each diluted at 1:5,000, were used as primary antibodies.

As a result, it was confirmed that the levels of cleaved PARP and cleaved caspase-3 in the HMC-1.2 cell line treated with PHI-101 were higher than those in the HMC-1.2 cell lines treated with the control compounds, indicating that PHI-101 had an apoptotic effect against the mastocytosis cell line (FIG. 8).

**Example 3. Evaluation of inhibitory activity of PHI-101 against mastocytosis in mouse model**

*3-1. Establishment of mastocytosis mouse model using HMC-1.2 cell line*

As mice, 6-8-week-old NOD SCID female mice (18 to 22g) (GemPharmatech Co., Ltd.) were prepared. Mice were acclimatized for 7 days before use in the experiment, and mice with abnormal health were excluded from the experiment. Mice were bred in a cage (300 x 180 x 150 mm) at a 22±3°C and a relative humidity of 40% to 70% with a 12-hr light/12-hr dark cycle. Mice were allowed access to sterilized dry granular food (Beijing Keaoxieli Feed Co., Ltd., Beijing, China) and water *ad libitum* for the entire experimental period except for the period specified in the protocol.

50 ml of HMC-1.2 cells suspension-cultured in a 75T flask were placed in a conical tube and centrifuged at 1,200 rpm for 2 minutes, centrifuged with DPBS (Dulbecco's Phosphate-Buffered Saline, Welgene) at 1,200 rpm for 2 minutes, washed, and then suspended in RPMI1640 (Welgene) without antibiotics and fetal bovine serum (FBS). Then, the cells were stained with trypan blue, counted with a hemocytometer, and then HMC-1.2 cells (1 x $10^6$) in a mixture of 0.1 ml L-15 and Matrigel (BD, cat. No. 356234) (1:1) were subcutaneously injected into the right flanks of mice anesthetized by intraperitoneal administration of Avertin (250 mg/kg), thus inducing tumors.

Thereafter, when the average tumor volume reached 150 to 200 $mm^3$, 4 mice were assigned to each group so that the average tumor volume at each group and treatment time was the same between the groups. Then, each of imatinib at a dose of 90 mg/kg, sunitinib at doses of 20 and 40 mg/kg, and PHI-101 at doses of 7, 20, 40 and 80 mg/kg was administered orally to the mice once daily for 3 weeks. For oral administration, each compound was dissolved in 5% 1-methyl-2-pyrrolidone and then completely mixed in 15% Kolliphor, 30% PEG E400 HS15 and 50% 0.05M citric acid.

*3-2. Evaluation of mastocytosis inhibitory activity*

**In** the mastocytosis mouse model with the HMC-1.2 cell line, whether mastocytosis was inhibited by administration of PHI-101 was examined.

Specifically, while the presence or absence of abnormalities in the mastocytosis mouse model of Example 3-1 was monitored daily by checking the skin condition, bleeding and stool status, whether the body weight changed was checked by measuring the mouse body weight at intervals of 3 days after the start of oral administration of each drug. In addition, the width and length of the tumor were measured using a caliper at 3-day intervals, and the tumor volume was calculated using the following equation.

$$V = (W(2) \times L)/2$$

(V: tumor volume, W: tumor width, and L: tumor length)

In addition, the mice were sacrificed 3 weeks after the start of oral administration of each drug and the tumor weight was measured using a digital scale. For each data, one-way ANOVA was used to test the significance of each group.

As a result, it was confirmed that the growth of mastocytosis cells was inhibited by the administration of PHI-101 in a concentration-dependent manner, and that the growth of mastocytosis cells in the group treated with PHI-101 at a concentration of 40 mg/kg or more was significantly inhibited compared to that in the group treated with each control compound (FIGS. 9 to 12).

[Table 3]

| | Dose (mpk, mg/kg) | Tumor growth inhibition (TGI) (%) | |
|---|---|---|---|
| | | Timor volume | Tumor weight |
| Vehicle | - | 0 | 0 |
| Imatinib | 90 | 11.3 | 7.8 |
| Sunitinib | 20 | 37.3 | 49.5 |
| | 40 | 46.2 | 47.9 |

(continued)

| | Dose (mpk, mg/kg) | Tumor growth inhibition (TGI) (%) | |
|---|---|---|---|
| | | Timor volume | Tumor weight |
| PHI-101 | 7 | 30.2 | 32.7 |
| | 20 | 43.6 | 51.6 |
| | 40 | 68.4 | 68.3 |
| | 80 | 78.5 | 82.7 |

Meanwhile, no abnormal symptoms were found in the mastocytosis mouse model during the experiment, and it was confirmed that there was no administration group that showed non-specific weight loss (<10%), and PHI-101 caused no weight loss even at a high dose of 80 mg/kg.

*3-3. Evaluation of inhibitory activity against inflammatory response caused by mastocytosis*

**In** the mastocytosis mouse model with the HMC-1.2 cell line, whether an inflammatory response caused by mastocytosis was inhibited by the administration of PHI-101 was examined.

Specifically, 3 weeks after administering each drug to the mastocytosis mouse model of Example 3-1, the mice were sacrificed, the body weight and spleen weight were measured using a digital scale, and then the spleen/body weight ratio was calculated.

**As** a result, it was confirmed that the administration of PHI-101 decreased the spleen/body weight ratio in a concentration-dependent manner compared to that in the control group (FIGS. 13 and 14 and Table 4), indicating that the inflammation response caused by mastocytosis was also effectively inhibited by the administration of PHI-101.

[Table 4]

| | Dose (mpk, mg/kg) | Spleen/body weight ratio | |
|---|---|---|---|
| | | % of control | Inhibition (%) |
| Vehicle | - | 100.0 | 0 |
| Imatinib | 90 | 97.7 | 2.3 |
| Sunitinib | 20 | 104.3 | -4.3 |
| | 40 | 102.7 | -2.7 |
| PHI-101 | 7 | 105.0 | -5 |
| | 20 | 95.0 | 5 |
| | 40 | 55.7 | 44.3 |
| | 80 | 46.3 | 53.7 |

**Example 4. *In vitro* examination of inhibitory effect of PHI-101 against binding activity of various c-kit protein mutations**

*4-1. Kinase assay*

A kinase-tagged T7 phage strain was prepared from an *E. coli* host derived from a BL21 strain. *E. coli* was grown in log-phase, infected with T7 phage, and then incubated with shaking at 32°C until lysis. The lysates were centrifuged and filtered to remove cell debris. Thereafter, the kinase produced in HEK-293 cells was labeled with DNA for qPCR detection. Streptavidin-coated magnetic beads were treated with biotinylated small molecule ligand for 30 minutes at room temperature to produce affinity resins for kinase assay. The liganded beads were blocked with excess biotin and washed with blocking buffer (SeaBlock(Pierce), 1% BSA, 0.05% Tween 20, 1 mM DTT) to remove unbound ligand and to reduce nonspecific binding. Binding reactions were induced by combining kinase, ligand affinity beads and PHI-101 in 1x binding buffer (20% SeaBlock, 0.17x PBS, 0.05% Tween 20, 6 mM DTT). PHI-101 was prepared as a 111X stock in 100% DMSO. The binding constant (Kd) was determined using an 11-point 3-fold compound dilution series with three DMSO control points. All compounds for Kd measurements were dispensed by acoustic transfer (non-contact dispensing) in 100%

DMSO. The compounds were then diluted directly into the assays such that the final concentration of DMSO was 0.9%. All reactions were performed in polypropylene 384-well plates at a final volume of 0.02 ml. The assay plates were incubated at room temperature with shaking for 1 hour and the affinity beads were washed with wash buffer (lx PBS, 0.05% Tween 20). The beads were then re-suspended in elution buffer (lx PBS, 0.05% Tween 20, 0.5 μM non-biotinylated affinity ligand) and incubated at room temperature with shaking for 30 minutes. The kinase concentration in the eluates was measured by qPCR.

*4-2. Compound treatment*

11-point 3-fold serial dilutions of each test compound were prepared in 100% DMSO at 100x final test concentration and then diluted to 1x in the assay (final DMSO concentration=1%). Most Kd values were determined using a compound top concentration = 30,000 nM. If the initial Kd determined was <0.5 nM (lowest concentration tested), the measurement was repeated with a serial dilution starting at a lower top concentration. A $K_d$ value reported as 40,000nM indicates that the $K_d$ was determined to be >30,000nM.

*4-3. Binding constant analysis*

The Kd value of c-kit protein was calculated with a standard dose-response curve (Equation 1) using the Hill equation.

$$\text{Response} = \text{Backgroud} + \frac{Signal - Background}{1 + (Kd^{Hill\ Slope} / Dose^{Hill\ Slope})}$$

The Hill Slope was set to -1. Curves were fitted using a non-linear least square fit with the Levenberg-Marquardt algorithm.

*4-4. Evaluation of inhibitory effect of PHI-101 against binding activity of various c-kit protein mutations*

The inhibitory effect of PHI-101 against the binding activity of various c-kit protein mutations was analyzed by the method described in Examples 4-1 to 4-3.

As a result, it was confirmed that PHI-101 inhibited the binding activity of seven mutant c-kit proteins (Table 5).

[Table 5]

| KIT mutation | Kd (nM) |
|---|---|
| KIT (A829P) | 1.3 |
| KIT (D816H) | 4.8 |
| KIT (D816V) | 2.1 |
| KIT (L576P) | 39 |
| KIT (V559D) | 16 |
| KIT (V559D, T670I) | 27 |
| KIT (V559D, V654A) | 26 |

From the above results, it was confirmed that PHI-101 can be particularly effectively used for the treatment of mastocytosis having A829P, D816H, D816V, L576P or V559D mutation in c-kit protein, mastocytosis having both V559D and T670I mutations in c-kit protein, and mastocytosis having both V559D and V654A mutations in c-kit protein.

## Claims

**1.** A compound represented by the following Formula 1 or a pharmaceutically acceptable salt thereof:

[Formula 1]

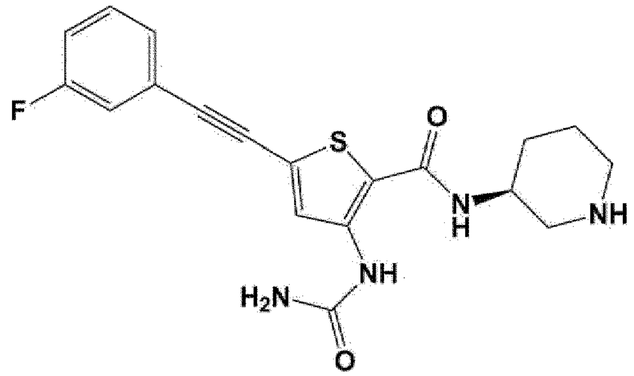

for use in preventing or treating mastocytosis.

2. The compound for use of claim 1, wherein the mastocytosis is any one disease selected from the group consisting of mast cell leukemia, systemic mastocytosis, and invasive systemic mastocytosis.

3. The compound for use of claim 1, wherein the mastocytosis is caused by substitution of valine or histidine for aspartic acid at amino acid position 816 of c-kit protein.

4. The compound for use of claim 1, wherein the mastocytosis is caused by at least one substitution selected from the group consisting of:

substitution of valine for glycine at amino acid position 560 of c-kit protein;
substitution of proline for alanine at amino acid position 829 of c-kit protein;
substitution of proline for leucine at amino acid position 576 of c-kit protein;
substitution of aspartic acid for valine at amino acid position 559 of c-kit protein;
substitution of isoleucine for threonine at amino acid position 670 of c-kit protein; and
substitution of alanine for valine at amino acid position 654 of c-kit protein

5. The compound for use of any one of claims 1-4, wherein the compound is contained in a pharmaceutical composition or food composition.

**Patentansprüche**

1. Verbindung, die durch die folgende Formel 1 dargestellt wird, oder ein pharmazeutisch verträgliches Salz davon:

[Formel 1]

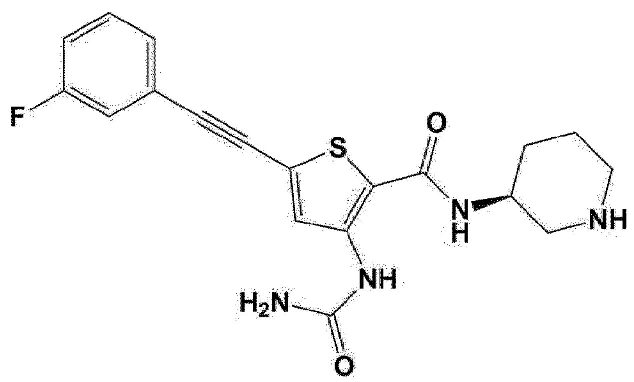

zur Vorbeugung oder Behandlung von Mastozytose.

2. Verbindung zur Verwendung nach Anspruch 1, wobei die Mastozytose eine beliebige Krankheit ist, die aus der

Gruppe ausgewählt ist, die aus Mastzellleukämie, systemischer Mastozytose und invasiver systemischer Mastozytose besteht.

3. Verbindung zur Verwendung nach Anspruch 1, wobei die Mastozytose durch Substitution von Asparaginsäure durch Valin oder Histidin an der Aminosäureposition 816 des c-kit-Proteins verursacht ist.

4. Verbindung zur Verwendung nach Anspruch 1, wobei die Mastozytose durch mindestens eine Substitution verursacht ist, ausgewählt aus der Gruppe bestehend aus:

   Substitution von Glycin durch Valin an der Aminosäureposition 560 des c-kit-Proteins;
   Substitution von Alanin durch Prolin an der Aminosäureposition 829 des c-kit-Proteins;
   Substitution von Leucin durch Prolin an der Aminosäureposition 576 des c-kit-Proteins;
   Substitution von Valin durch Asparaginsäure an der Aminosäureposition 559 des c-kit-Proteins;
   Substitution von Threonin durch Isoleucin an der Aminosäureposition 670 des c-kit-Proteins; und
   Substitution von Valin durch Alanin an der Aminosäureposition 654 des c-kit-Proteins.

5. Verbindung zur Verwendung nach einem der Ansprüche 1-4, wobei die Verbindung in einer pharmazeutischen Zusammensetzung oder einer Nahrungsmittelzusammensetzung enthalten ist.

## Revendications

1. Composé représenté par la formule 1 suivante ou sel pharmaceutiquement acceptable de celui-ci :

[formule 1]

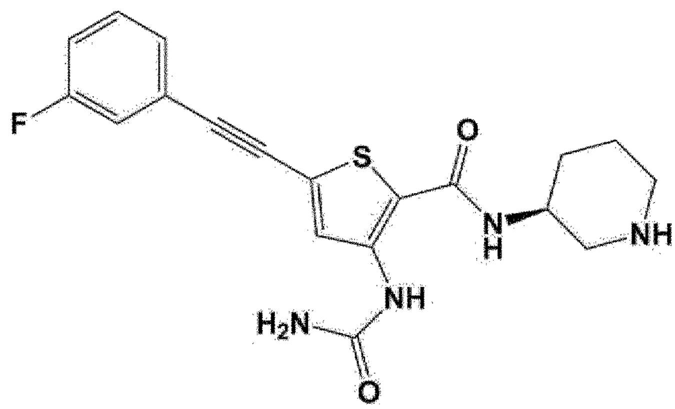

à utiliser pour la prévention ou le traitement de la mastocytose.

2. Composé selon la revendication 1,
dans lequel la mastocytose est une maladie choisie dans le groupe comprenant la leucémie à mastocytes, la mastocytose systémique et la mastocytose systémique invasive.

3. Composé selon la revendication 1,
dans lequel la mastocytose est provoquée par la substitution de valine ou d'histidine pour l'acide aspartique à la position d'acide aminé 816 de la protéine c-kit.

4. Composé selon la revendication 1,
dans lequel la mastocytose est provoquée par au moins une substitution choisie dans le groupe comprenant :

   la substitution de valine pour la glycine à la position d'acide aminé 560 de la protéine c-kit ;
   la substitution de proline pour l'alanine à la position d'acide aminé 829 de la protéine c-kit ;
   la substitution de proline pour la leucine à la position d'acide aminé 576 de la protéine c-kit ;

la substitution d'acide aspartique pour la valine à la position d'acide aminé 559 de la protéine c-kit ;
la substitution d'isoleucine pour la thréonine à la position d'acide aminé 670 de la protéine c-kit ; et
la substitution d'alanine pour la valine à la position d'acide aminé 654 de la protéine c-kit.

**5.** Composé selon l'une des revendications 1 à 4,
le composé étant contenu dans une composition pharmaceutique ou dans une composition alimentaire.

Fig. 1

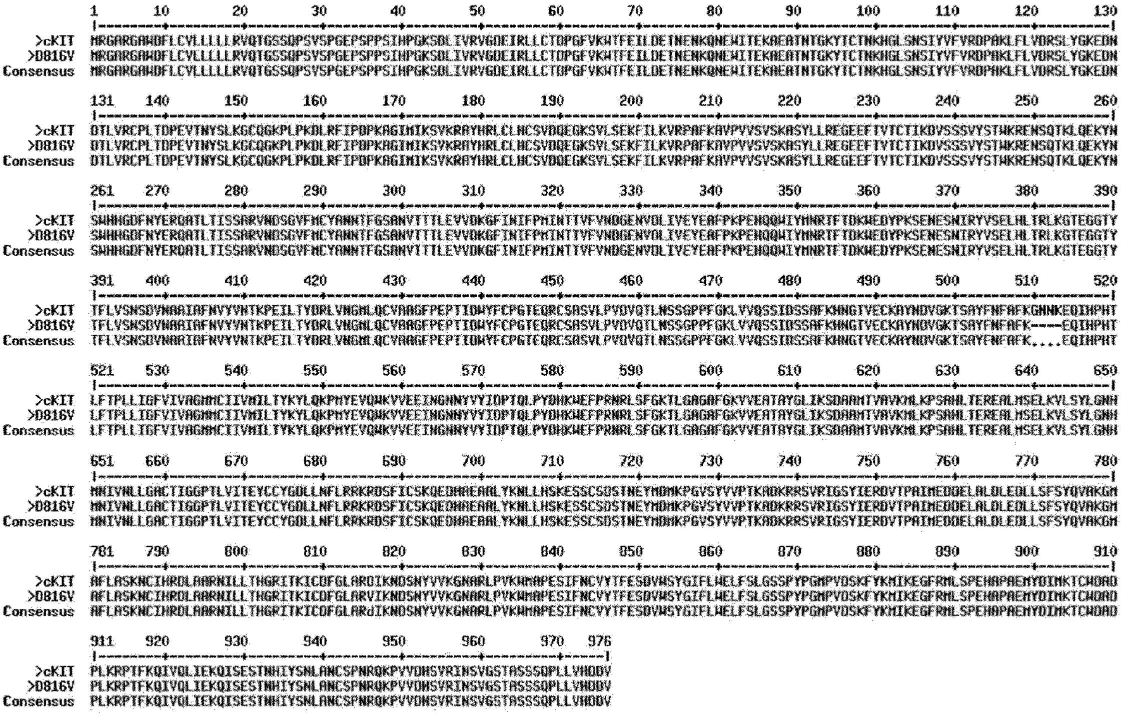

Fig. 2

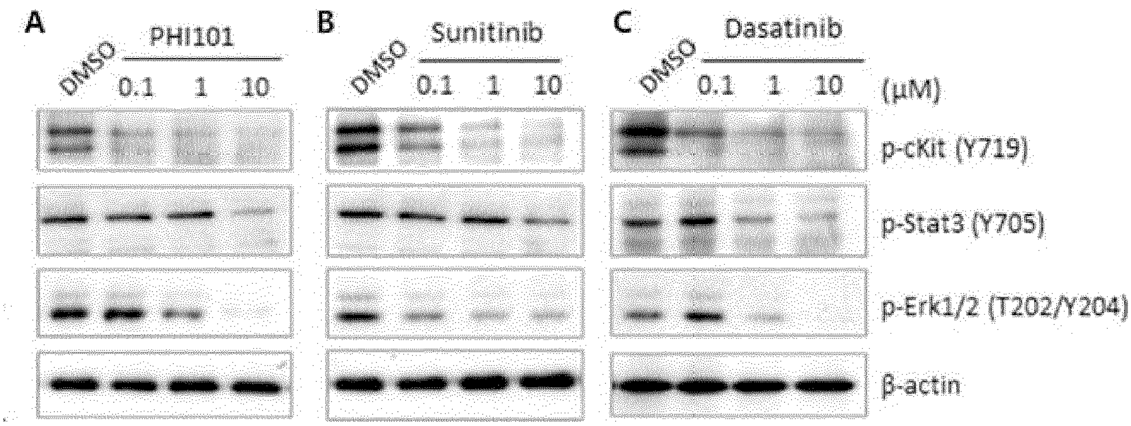
A
PHI101
DMSO
0.1
1
10
B
Sunitinib
DMSO
0.1
1
10
C
Dasatinib
DMSO
0.1
1
10
(µM)
p-cKit (Y719)
p-Stat3 (Y705)
p-Erk1/2 (T202/Y204)
β-actin

Fig. 3

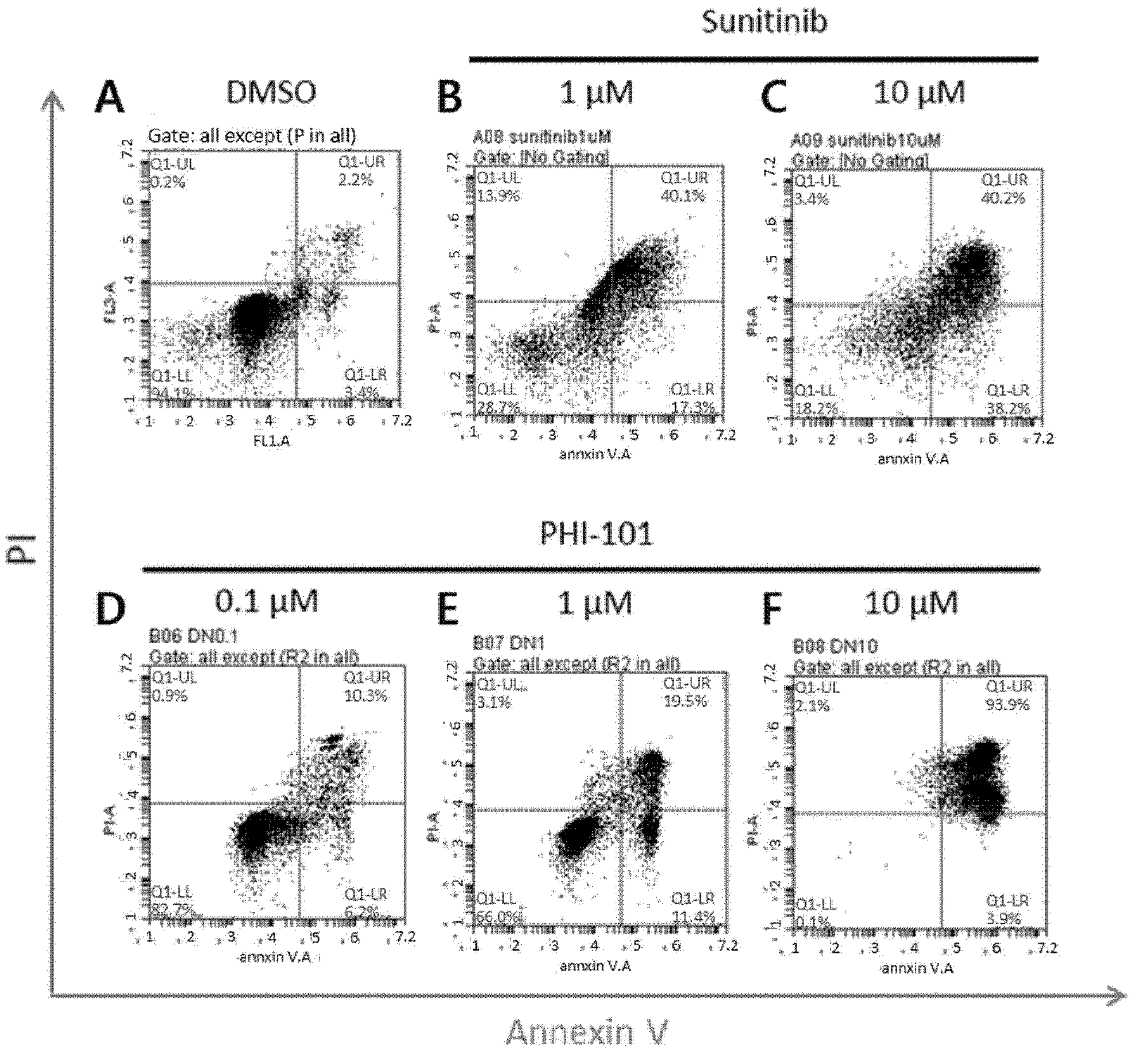
Sunitinib
A
DMSO
B
1 µM
C
10 µM
Gate: all except (P in all)
A08 sunitinib1uM
Gate: [No Gating]
A09 sunitinib10uM
Gate: [No Gating]
Q1-UL 0.2%
Q1-UR 2.2%
Q1-LL 94.1%
Q1-LR 3.4%
Q1-UL 13.9%
Q1-UR 40.1%
Q1-LL 28.7%
Q1-LR 17.3%
Q1-UL 3.4%
Q1-UR 40.2%
Q1-LL 18.2%
Q1-LR 38.2%
FL3.A
FL1.A
PI.A
annxin V.A
PI
PHI-101
D
0.1 µM
E
1 µM
F
10 µM
B06 DN0.1
Gate: all except (R2 in all)
B07 DN1
Gate: all except (R2 in all)
B08 DN10
Gate: all except (R2 in all)
Q1-UL 0.9%
Q1-UR 10.3%
Q1-LL 82.7%
Q1-LR 6.2%
Q1-UL 3.1%
Q1-UR 19.5%
Q1-LL 66.0%
Q1-LR 11.4%
Q1-UL 2.1%
Q1-UR 93.9%
Q1-LL 0.1%
Q1-LR 3.9%
Annexin V

Fig. 4

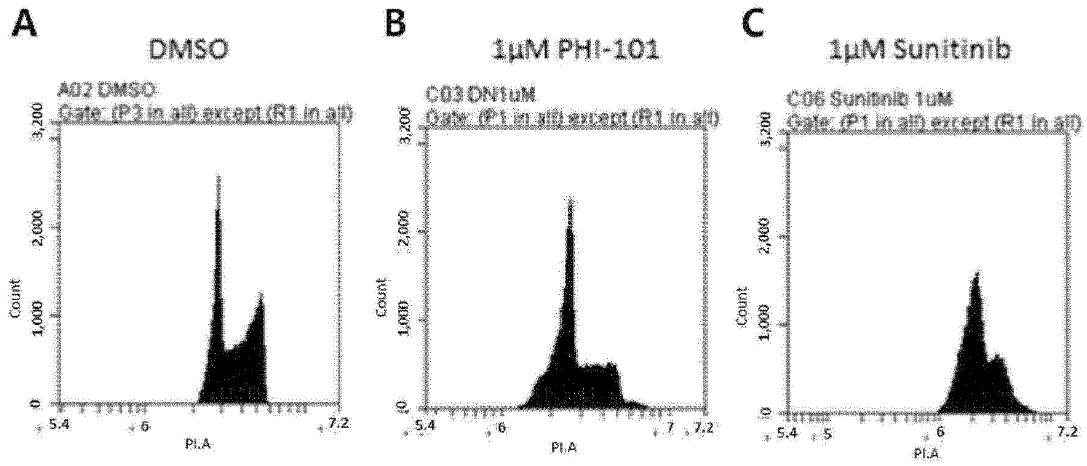
A
DMSO
A02 DMSO
Gate: (P3 in all) except (R1 in all)
B
1μM PHI-101
C03 DN1uM
Gate: (P1 in all) except (R1 in all)
C
1μM Sunitinib
C06 Sunitinib 1uM
Gate: (P1 in all) except (R1 in all)
Count
PI.A


Fig. 5

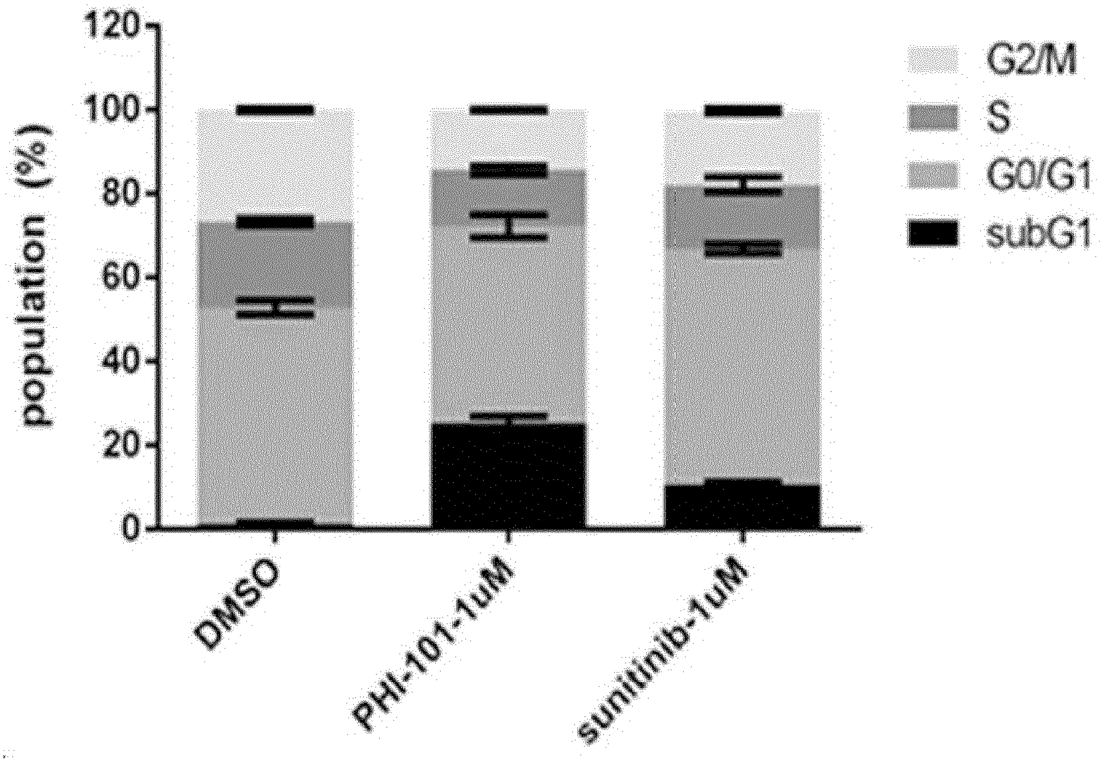
population (%)
G2/M
S
G0/G1
subG1
DMSO
PHI-101-1uM
sunitinib-1uM

Fig. 6

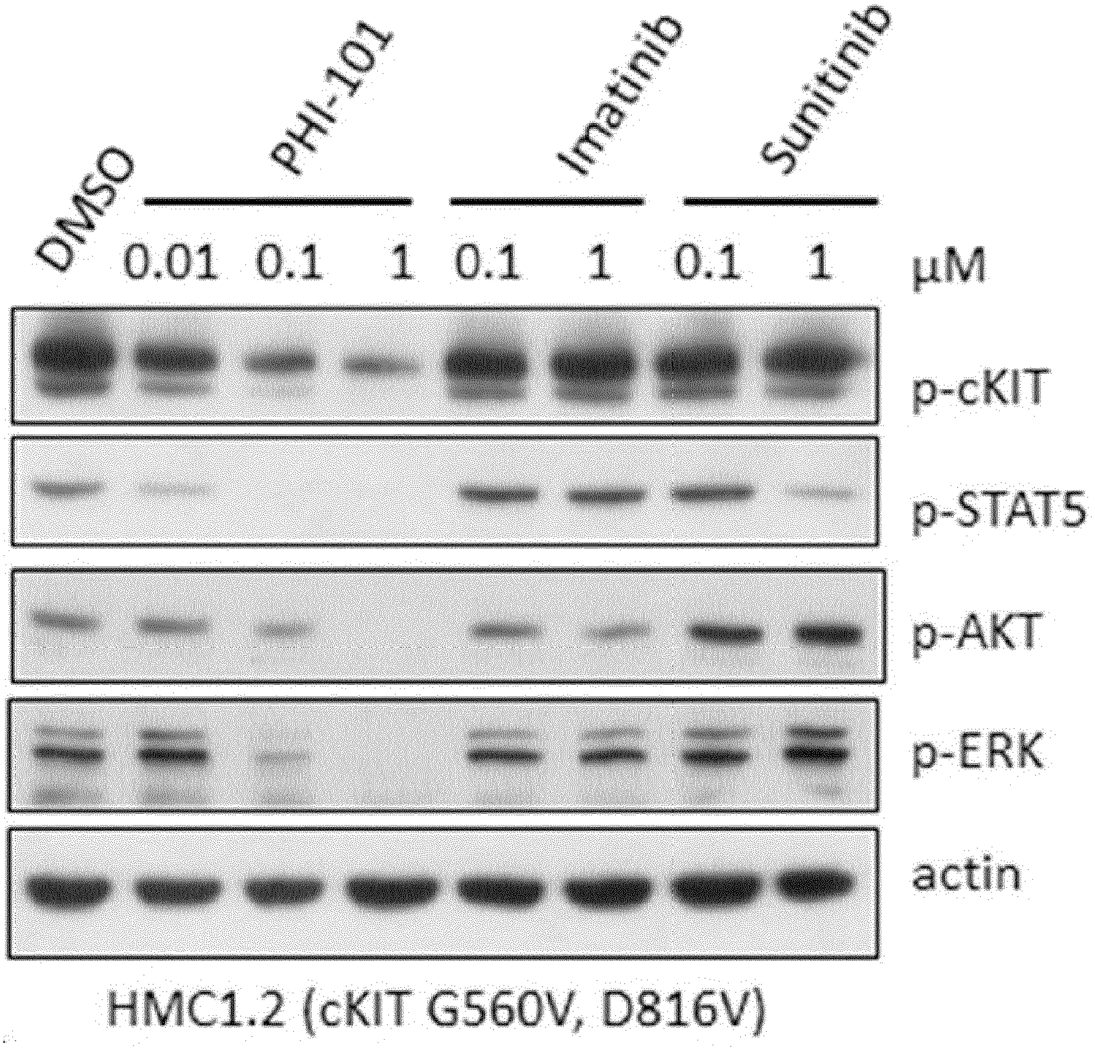
DMSO
PHI-101
Imatinib
Sunitinib
0.01
0.1
1
0.1
1
0.1
1
μM
p-cKIT
p-STAT5
p-AKT
p-ERK
actin
HMC1.2 (cKIT G560V, D816V)

Fig. 7

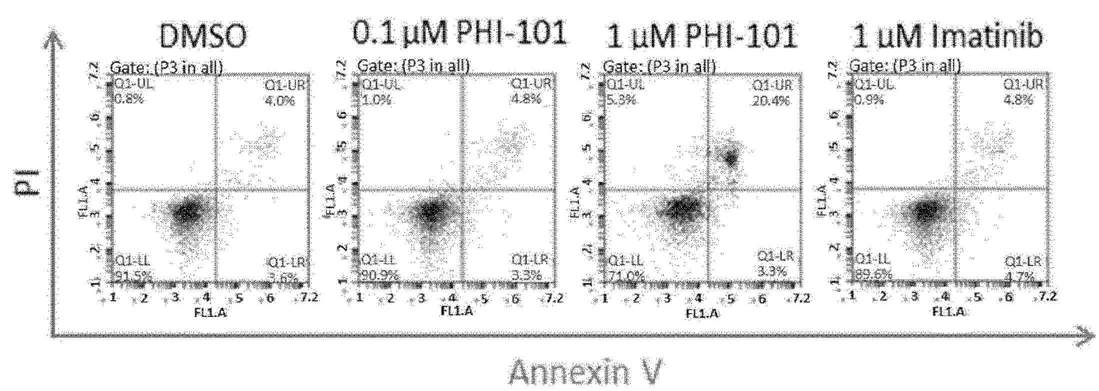
DMSO
0.1 μM PHI-101
1 μM PHI-101
1 uM Imatinib
PI
Annexin V
Gate: (P3 in all)
Q1-UL 0.8%
Q1-UR 4.0%
Q1-LL 91.5%
Q1-LR 3.6%
Q1-UL 1.0%
Q1-UR 4.8%
Q1-LL 90.9%
Q1-LR 3.3%
Q1-UL 5.3%
Q1-UR 20.4%
Q1-LL 71.0%
Q1-LR 3.3%
Q1-UL 0.9%
Q1-UR 4.8%
Q1-LL 89.6%
Q1-LR 4.7%
FL1.A

Fig. 8

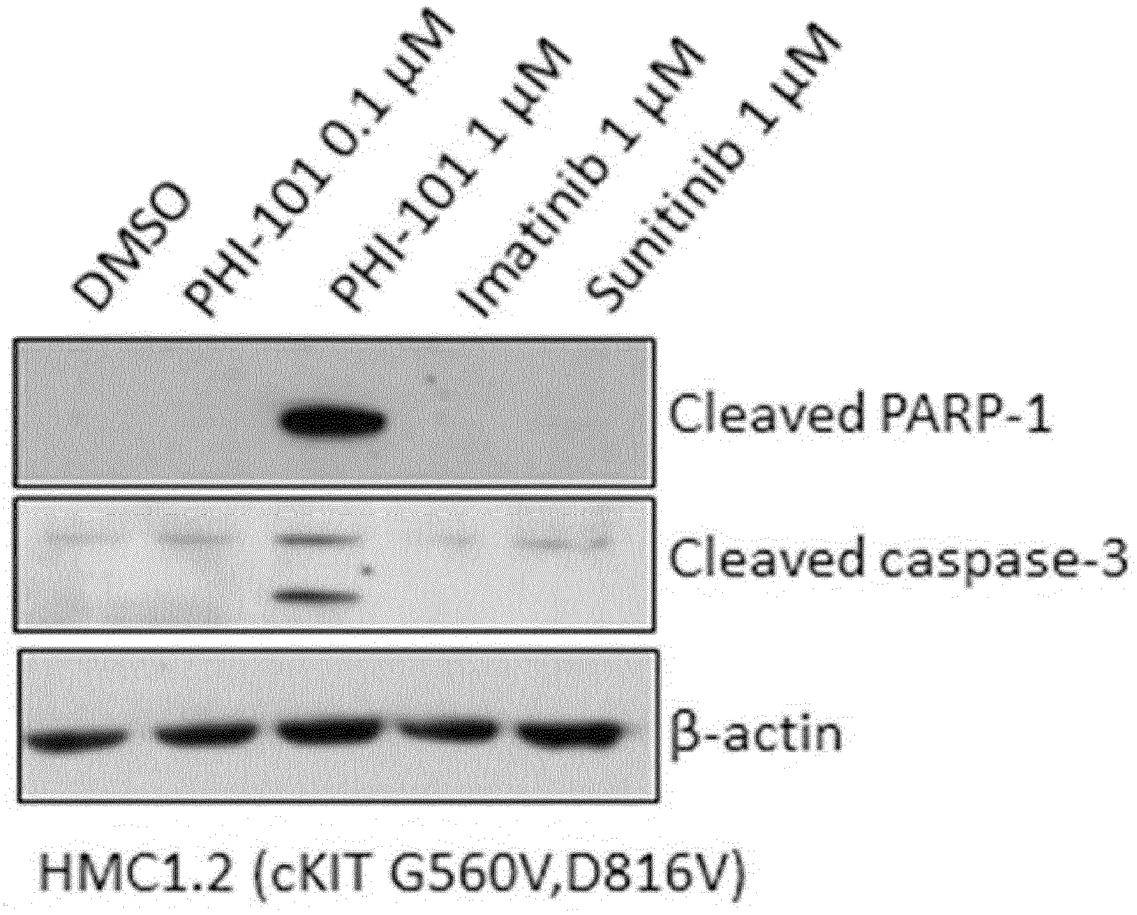
DMSO
PHI-101 0.1 μM
PHI-101 1 μM
Imatinib 1 μM
Sunitinib 1 μM
Cleaved PARP-1
Cleaved caspase-3
β-actin
HMC1.2 (cKIT G560V,D816V)

Fig. 9

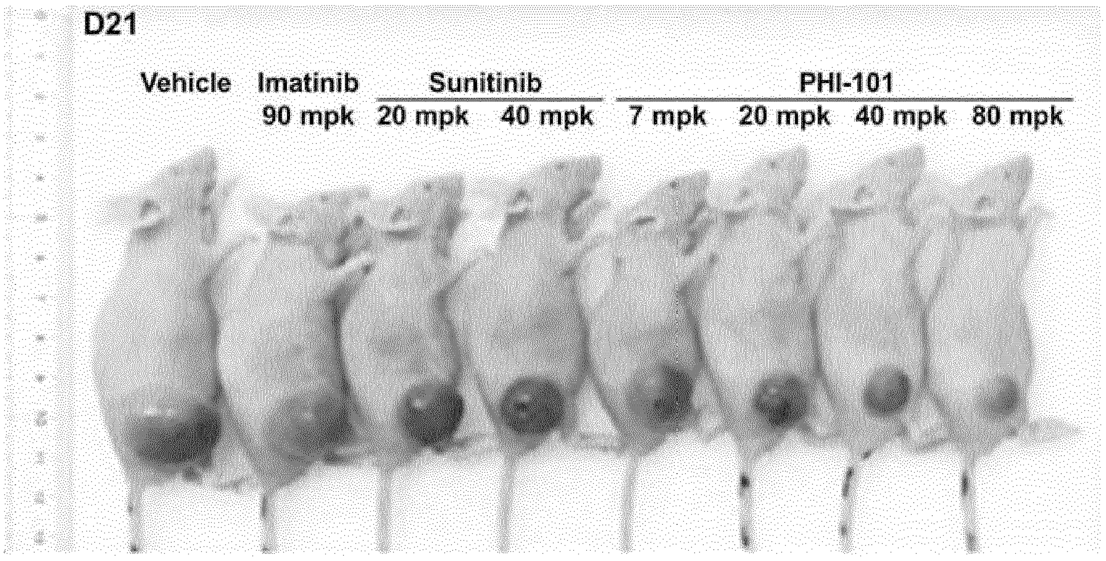
D21
Vehicle
Imatinib
90 mpk
Sunitinib
20 mpk
40 mpk
PHI-101
7 mpk
20 mpk
40 mpk
80 mpk

Fig. 10

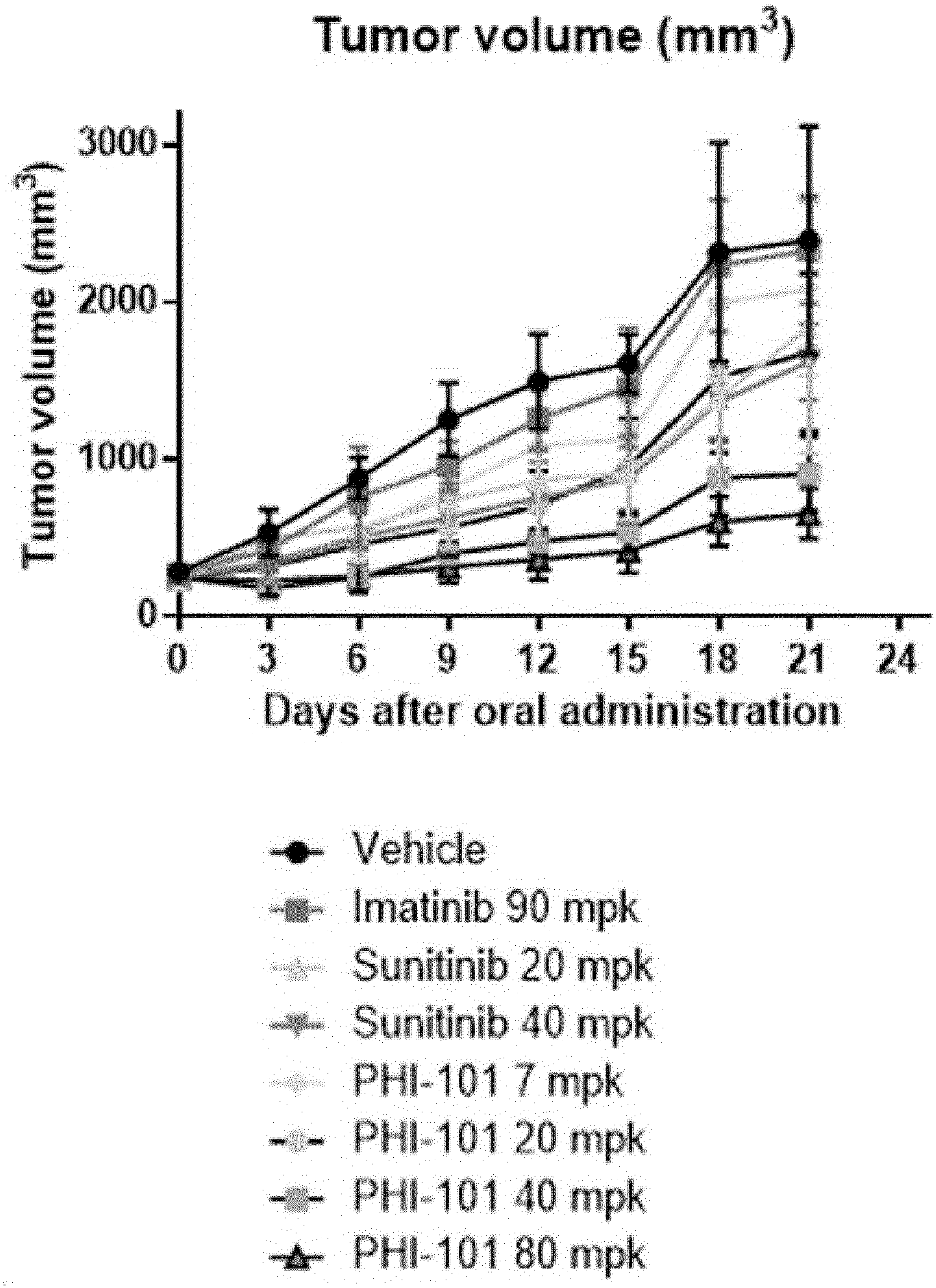
Tumor volume (mm3)
Tumor volume (mm3)
3000
2000
1000
0
0
3
6
9
12
15
18
21
24
Days after oral administration
Vehicle
Imatinib 90 mpk
Sunitinib 20 mpk
Sunitinib 40 mpk
PHI-101 7 mpk
PHI-101 20 mpk
PHI-101 40 mpk
PHI-101 80 mpk

Fig. 11

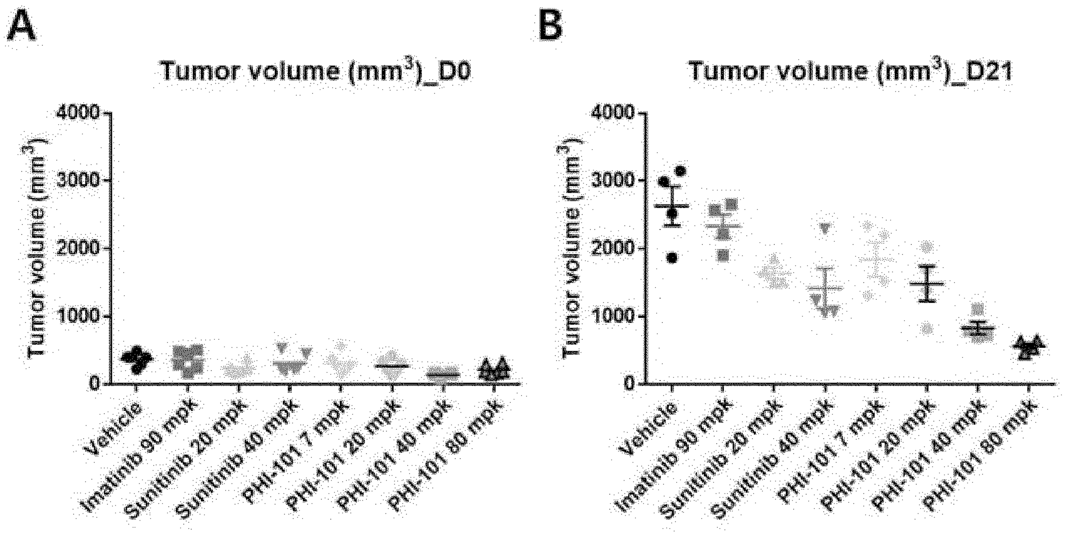
A
Tumor volume (mm$^3$)_D0
Tumor volume (mm$^3$)
4000
3000
2000
1000
0
Vehicle
Imatinib 90 mpk
Sunitinib 20 mpk
Sunitinib 40 mpk
PHI-101 7 mpk
PHI-101 20 mpk
PHI-101 40 mpk
PHI-101 80 mpk
B
Tumor volume (mm$^3$)_D21
Tumor volume (mm$^3$)
4000
3000
2000
1000
0
Vehicle
Imatinib 90 mpk
Sunitinib 20 mpk
Sunitinib 40 mpk
PHI-101 7 mpk
PHI-101 20 mpk
PHI-101 40 mpk
PHI-101 80 mpk

Fig. 12

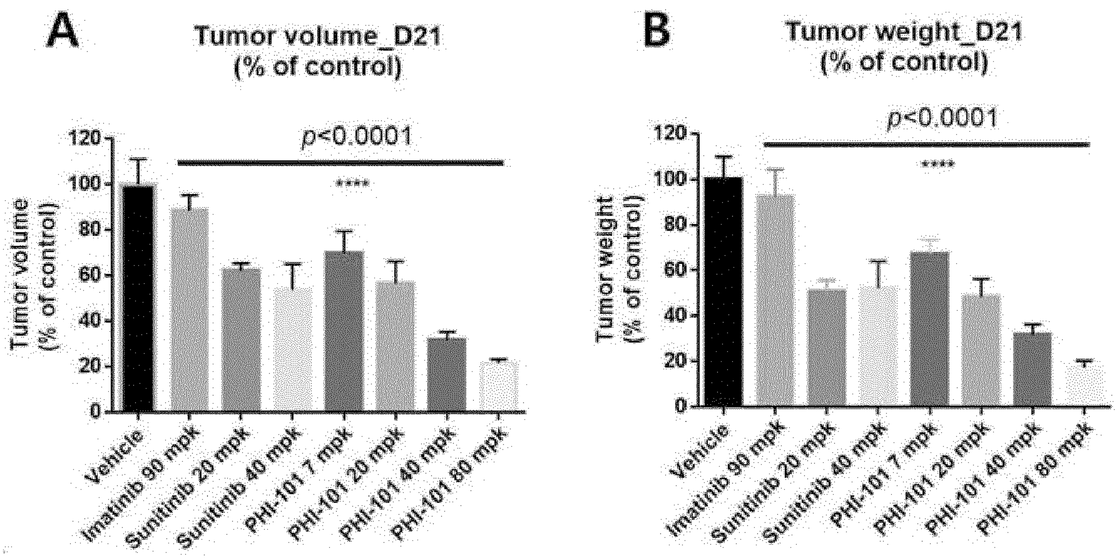
A
Tumor volume_D21
(% of control)
p<0.0001
****
Tumor volume
(% of control)
120
100
80
60
40
20
0
Vehicle
Imatinib 90 mpk
Sunitinib 20 mpk
Sunitinib 40 mpk
PHI-101 7 mpk
PHI-101 20 mpk
PHI-101 40 mpk
PHI-101 80 mpk
B
Tumor weight_D21
(% of control)
p<0.0001
****
Tumor weight
(% of control)
120
100
80
60
40
20
0
Vehicle
Imatinib 90 mpk
Sunitinib 20 mpk
Sunitinib 40 mpk
PHI-101 7 mpk
PHI-101 20 mpk
PHI-101 40 mpk
PHI-101 80 mpk

Fig. 13

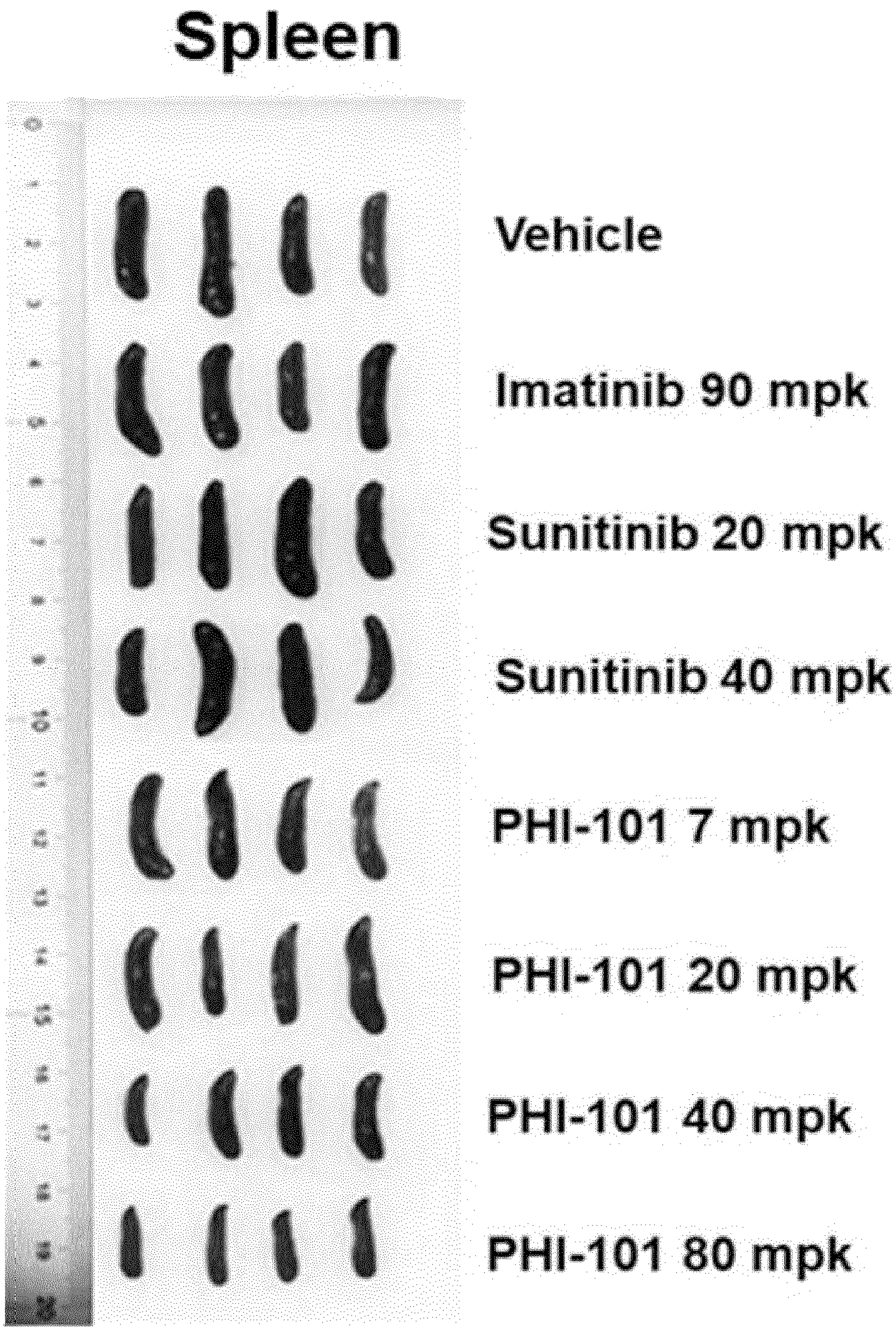
Spleen
Vehicle
Imatinib 90 mpk
Sunitinib 20 mpk
Sunitinib 40 mpk
PHI-101 7 mpk
PHI-101 20 mpk
PHI-101 40 mpk
PHI-101 80 mpk

Fig. 14

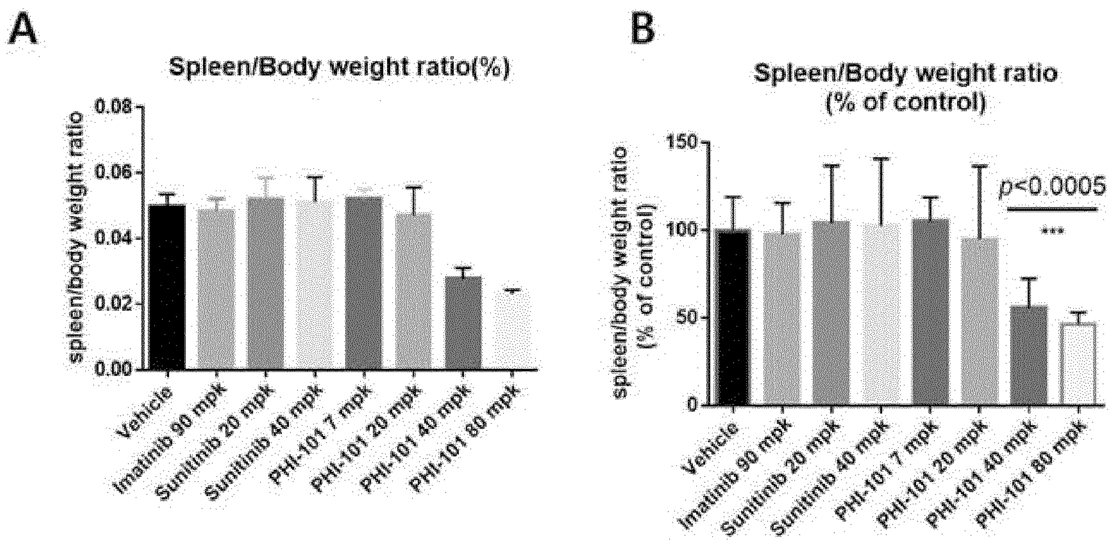
A
Spleen/Body weight ratio(%)
spleen/body weight ratio
0.08
0.06
0.04
0.02
0.00
Vehicle
Imatinib 90 mpk
Sunitinib 20 mpk
Sunitinib 40 mpk
PHI-101 7 mpk
PHI-101 20 mpk
PHI-101 40 mpk
PHI-101 80 mpk
B
Spleen/Body weight ratio
(% of control)
spleen/body weight ratio
(% of control)
150
100
50
0
p<0.0005
***
Vehicle
Imatinib 90 mpk
Sunitinib 20 mpk
Sunitinib 40 mpk
PHI-101 7 mpk
PHI-101 20 mpk
PHI-101 40 mpk
PHI-101 80 mpk

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3418275 A
- US 2006063773 A


### Non-patent literature cited in the description


- **AKIN C** ; **BROCKOW K** ; **D'AMBROSIO C et al.** Effects of tyrosine kinase inhibitor STI571 on human mast cells bearing wild-type or mutated forms of c-kit. *Exp Hematol*, 2003, vol. 31, 686-692
- **MA Y** ; **ZENG S** ; **METCALFE DD et al.** The c-KIT mutation causing human mastocytosis is resistant to STI571 and other KIT kinase inhibitors; kinases with enzymatic site mutations show different inhibitor sensitivity profiles than wild-type kinases and those with regulatory type mutations. *Blood*, 2002, vol. 99, 1741-1744
- **FROST MJ** ; **FERRAO PT** ; **HUGHES TP** ; **ASHMAN LK**. Juxtamembrane mutant V560GKit is more sensitive to Imatinib (STI571) compared with wild-type c-kit whereas the kinase domain mutant D816VKit is resistant. *Mol Cancer Ther.*, 2002, vol. 1, 1115-1124
- **AKIN C** ; **FUMO G** ; **YAVUZ AS** ; **LIPSKY PE** ; **NECKERS L** ; **METCALFE DD**. A novel form of mastocytosis associated with a transmembrane c-kit mutation and response to imatinib. *Blood.*, 2004, vol. 103, 3222-3225
- **GALLOGLY MM. et al.** *Therapeutic Advances in Hematology*, 2017, vol. 8 (9), 245-261